# FASCICULE DE BREVET EUROPEEN

(11) **EP 3 586 119 B1**
(45) Date de publication et mention de la délivrance du brevet: **04.11.2026**
(21) Numéro de dépôt: 18710094.6
(22) Date de dépôt: 20.02.2018
(51) Int. Cl.: G01N 27/48

(54) **DISPOSITIF ET METHODE ELECTROCHIMIQUE POUR LA MESURE DES DIFFERENTES FORMES NON COMPLEXEES DU DIOXYDE DE SOUFRE DANS UN MILIEU LIQUIDE AQUEUX**
VORRICHTUNG UND VERFAHREN ZUR MESSUNG NICHT-KOMPLEXIERTER FORMEN VON SCHWEFELDIOXID IN EINEM FLÜSSIGEN, WÄSSRIGEN MEDIUM
ELECTROCHEMICAL DEVICE AND METHOD FOR MEASURING DIFFERENT NON-COMPLEXED FORMS OF SULFUR DIOXIDE IN A LIQUID AQUEOUS MEDIUM

(30) Priorité: 21.02.2017 FR 1751370
(43) Date de publication de la demande: 01.01.2020
(73) Titulaire: Université de Bordeaux, 33000 Bordeaux (FR); Institut Polytechnique de Bordeaux, 33402 Talence Cedex (FR); Centre National de la Recherche Scientifique, 75016 Paris (FR)
(72) Inventeur: ARBAULT, Stéphane, 33170 Gradignan (FR); RECULUSA, Stéphane, 33600 Pessac (FR); KUHN, Alexander, 33420 Guillac (FR); SOJIC, Neso, 33610 Cestas (FR); MASSE, Pascal, 33450 Izon (FR)
(74) Mandataire: Santarelli
(86) Numéro de dépôt international: PCT/FR2018/050397
(87) Numéro de publication internationale: WO 2018/154226

(56) Documents cités:
- US-A1- 2015 184 117
- BALDO M ANTONIETTA ET AL: "Voltammetric Determination of Free Sulfur Dioxide in Wines Using Platinum and Gold Disc M icroelect rodes*", ANALYST, 1 June 1994 (1994-06-01), pages 1239 - 1244, XP093065762, Retrieved from the Internet <URL:https://pubs.rsc.org/en/content/articlepdf/1994/an/an9941901239> [retrieved on 20230720]
- SCHNEIDER MARION ET AL: "Determination of the wine preservative sulphur dioxide with cyclic voltammetry using inkjet printed electrodes", FOOD CHEMISTRY, vol. 159, 18 March 2014 (2014-03-18), pages 428 - 432, XP028638730, ISSN: 0308-8146, DOI: 10.1016/J.FOODCHEM.2014.03.049
- MAKHOTKINA O ET AL: "The use of cyclic voltammetry for wine analysis: Determination of polyphenols and free sulfur dioxide", ANALYTICA CHIMICA ACTA, ELSEVIER, AMSTERDAM, NL, vol. 668, no. 2, 4 June 2010 (2010-06-04), pages 155 - 165, XP027054318, ISSN: 0003-2670, [retrieved on 20100424]
- KIM JIEUN ET AL: "Electrochemical sensor applications of Pt supported porous gold electrode prepared using cellulose-filter", KOREAN JOURNAL OF CHEMICAL ENGINEERING, SPRINGER NEW YORK LLC, US, KR, vol. 33, no. 1, 2 December 2015 (2015-12-02), pages 344 - 349, XP035948604, ISSN: 0256-1115, [retrieved on 20151202], DOI: 10.1007/S11814-015-0179-7
- SERHIY CHEREVKO ET AL: "Gold dissolution: towards understanding of noble metal corrosion", RSC ADVANCES, vol. 3, no. 37, 8 July 2013 (2013-07-08), pages 16516, XP055421175, DOI: 10.1039/c3ra42684j
- C. MONTES ET AL: "Critical Comparison between Modified Monier-Williams and Electrochemical Methods to Determine Sulfite in Aqueous Solutions", THE SCIENTIFIC WORLD JOURNAL, vol. 2012, 1 January 2012 (2012-01-01), pages 1 - 6, XP055421180, DOI: 10.1100/2012/168148

## Description

L'invention concerne une méthode et un dispositif électrochimique de détection et/ou quantification du dioxyde de soufre (SO₂) sous ses différentes formes non complexées (moléculaires et ioniques) dans un liquide alimentaire aqueux ou hydroalcoolique. Elle concerne également un procédé de régénération d'une électrode de mesure dont la surface active est entièrement en or.

Dans la suite de ce présent document, on désignera le SO₂ non complexé sous le terme « SO₂ libre ».

Du fait de ses propriétés antiseptiques, antioxydantes et antioxydasiques, le SO₂ est largement utilisé pour la conservation des fruits et dans l'élaboration de boissons aqueuses telles que des jus de fruits non fermentés, ou de boissons hydroalcooliques, telles que les vins effervescents ou non, champagnes, cidres, bières, jus de fruits fermentés.

L'ajout de SO₂ sous différentes formes chimiques, appelé sulfitage, a notamment lieu tout au long du processus d'élaboration du vin, de la vinification au conditionnement de manière à le protéger, l'aseptiser, et le conserver. Lors de l'incorporation de SO₂ dans un moût en fermentation ou dans un vin, une fraction de celui-ci va se combiner aux sucres, aux aldéhydes (principalement l'éthanal) ou à des cétones présents dans ce milieu riche en composés organiques. La fraction restante non complexée, dite libre, est celle qui possède les propriétés les plus intéressantes. La fraction la plus antiseptique du SO₂ libre est appelée SO₂ actif et correspond chimiquement au SO₂ moléculaire. La teneur en SO₂ actif est fonction du pH, de la température, du degré alcoolique et de la concentration en SO₂ libre. Elle est d'un grand intérêt œnologique car elle permet de traduire le niveau de protection du vin contre l'oxydation et la contamination de micro-organismes d'altération. Ainsi, le vinificateur a besoin de connaître la concentration en SO₂ libre tout au long de la vie du vin de manière à ajuster les quantités en SO₂. On appelle SO₂ libre le dioxyde de soufre présent sous les formes suivantes : H₂SO₃, HSO₃⁻ et SO₃²⁻, dont l'équilibre est fonction du pH et de la température :

| | |
|---|---|
| H₂SO₃ ⇆ H⁺+HSO₃⁻. | avec pK1 = 1,9 |
| HSO₃⁻ ⇆ H⁺+ SO₃²⁻. | avec pK2 = 7,2 |

Dans le vin, dont le pH est typiquement compris entre 3 et 4, les formes H₂SO₃ et HSO₃⁻ sont majoritaires.

A l'heure actuelle, deux méthodes sont utilisées par les vinificateurs pour doser le SO₂ libre présent dans le moût ou le vin.

La première méthode est appelée « méthode Ripper » et est décrite dans le recueil international des méthodes d'analyses - OIV-MA-AS323-04B.

Cette méthode couramment utilisée consiste en un dosage iodométrique du SO₂ libre en milieu acide et du SO₂ combiné après hydrolyse alcaline sur des échantillons de vins.

La seconde méthode est la méthode dite de « Franz Paul » et est décrite dans le recueil international des méthodes d'analyses - OIV-MA-AS323-04A.

Dans cette méthode, le H₂SO₃ formé par acidification du milieu est entrainé par un courant d'air ou d'azote ; il est fixé et oxydé en acide sulfurique (H₂SO₄) par barbotage dans une solution diluée neutre de peroxyde d'hydrogène.

L'H₂SO₄ ainsi formé est dosé par une solution titrée d'hydroxyde de sodium. Le SO₂ libre est extrait du vin par entrainement à froid (10°C environ) et le SO₂ combiné par entrainement à chaud.

Comme on le voit, ces méthodes d'analyse ne peuvent être mises en œuvre que par un laboratoire spécialisé et en dehors du site des exploitations viticoles.

Ces méthodes utilisent des mesures indirectes qui nécessitent un prélèvement d'échantillon ainsi qu'un ajout de réactifs. De ce fait, leurs protocoles sont complexes à mettre en œuvre.

Le document Schneider et al., Food Chemistry 159 (2014) pp. 428-432, décrit la détermination du dioxyde de soufre dans le vin par voltampérométrie cyclique au moyen d'électrodes imprimées par jet d'encre sur un film PET, dans une configuration à trois électrodes comprenant une électrode de référence Ag/AgCl et une contre-électrode en platine.

Ainsi, il existe un besoin pour une méthode et un dispositif de détection et/ou quantification rapide et direct du SO₂ libre, qui peuvent être mis en œuvre par un non spécialiste au sein des exploitations viticoles.

A cet effet, il est décrit une méthode électrochimique (non selon l'invention) pour la détection et/ou quantification du SO₂ libre dans un liquide alimentaire aqueux ou hydroalcoolique, caractérisée en ce qu'elle comprend :
a) l'introduction dans le liquide alimentaire de :
   - une électrode de mesure dont la surface active en contact avec le liquide alimentaire est entièrement en or,
   - une électrode de référence,
   - une contre-électrode, et
b) la mesure de la variation du courant produit par l'oxydation du SO₂ libre, présent dans le liquide alimentaire, lors d'un balayage en potentiel par voltampérométrie cyclique.

Un objet de la présente invention est une méthode électrochimique de détection et/ou quantification du dioxyde de soufre (SO₂) sous ses formes non complexées dites libres dans un liquide alimentaire aqueux ou hydroalcoolique caractérisé en ce qu'elle comprend :
a) l'introduction dans le liquide alimentaire d'une électrode de mesure dont la surface active en contact avec le liquide alimentaire est entièrement en or, cette électrode de mesure ayant un rapport R_{théo} entre son aire développée (A_{dev}) et son aire apparente (Aₐₚₚ) supérieur ou égal à 3,
b) la mesure de la variation du courant produit par l'oxydation du SO₂ libre, présent dans le liquide alimentaire, lors d'un balayage en potentiel par voltampérométrie cyclique effectué entre -1 V et +2 V, en utilisant une électrode de référence Ag/AgCl, à des vitesses de balayage en potentiel comprises entre 1 mV.s⁻¹ et 10000 mV.s⁻¹, de préférence entre 10 mV.s⁻¹ et 1000 mV.s⁻¹, plus préférablement comprises entre 10 mV.s⁻¹ et 100 mV.s⁻¹ ladite méthode comprend avant l'étape b), une étape b1) de balayage en potentiel, lors d'une voltampérométrie cyclique, effectuée entre +0,1 V et +1,5 V, en utilisant une électrode de référence Ag/AgCl, à des vitesses de balayage en potentiel comprises entre 1 mV.s⁻¹ et 10000 mV.s⁻¹, de préférence entre 10 mV.s⁻¹ et 1000 mV.s⁻¹, préférablement comprises entre 10 mV.s⁻¹ et 100 mV.s⁻1, sans mesure de la variation du courant.

Selon un mode de mise en œuvre de la méthode de l'invention, à l'étape b) le balayage en potentiel est effectué :
- entre +0,1 V et + 1,9 V, préférablement entre +0,1 V et +1,5 V,
- à des vitesses de balayage en potentiel comprises entre 10 mV.s⁻¹ et 1 000 mV.s⁻¹, plus préférablement comprises entre 10 mV.s⁻¹ et 100 mV.s⁻¹.

De préférence, le liquide alimentaire est du vin.

Selon un mode de mise en œuvre préféré de la méthode de l'invention, l'électrode de mesure a un rapport R_{théo} entre son aire développée (A_{dev}) et son aire apparente (Aₐₚₚ) compris entre 3 et 100, plus préférablement compris entre 3 et 20.

L'étape b1) telle que définie précédemment permettet l'oxydation puis la réduction de la surface active entièrement en or de l'électrode de mesure en contact avec le liquide alimentaire.

Selon toujours un mode de mise en œuvre de la méthode de l'invention, cette méthode comprend, de plus :
- avant l'étape b), une étape b2) d'établissement d'une abaque reliant la valeur du pH du liquide alimentaire en fonction de la position du pic de réduction des oxydes d'or lors de balayages en potentiel, par voltampérométrie cyclique, effectués dans le liquide alimentaire à différents pH, en utilisant une électrode Ag/AgCl comme électrode de référence, entre +1 V et +2 V, de préférence entre +0,1 V et +1,9 V, préférablement entre +0,1 V et +1,5 V, et à des vitesses de balayage en potentiel comprises entre 1 mV.s⁻¹ et 10 000 mV.s⁻¹, de préférence comprises entre 10 mV.s⁻¹ et 1 000 mV.s⁻¹, plus préférablement comprises entre 10 mV.s⁻¹ et 100 mV.s⁻¹,
- la mesure de la température T du liquide alimentaire, pendant l'étape b), et
- la détermination du titre alcoolique, TAV, du liquide alimentaire, ce par quoi on obtient le pourcentage de SO₂ moléculaire contenu dans le SO₂ libre, grâce à la formule suivante : $% {SO}_{2} moléculaire = 100 / \left[{10}^{\left(pH-pK1\right)}+1\right]$ dans laquelle ${pK}_{1} = 1 , 949 + \left(T-20\right) \times 0 , 0322 + \left(TAV-10\right) \times 0 , 01971$

Il est également décrit une méthode de régénération (non selon l'invention) d'une électrode de mesure dont la surface active est entièrement en or, caractérisée en ce qu'elle comprend :
a) l'introduction dans un liquide alimentaire de l'électrode dont la surface active est entièrement en or, et
b1) la mise en œuvre de balayage(s) en potentiel, lors d'une voltampérométrie cyclique, effectué(s) :
   - en utilisant une électrode Ag/AgCl comme électrode de référence,
   - entre +0,1 V et +1,5 V,
   - à des vitesses de balayage en potentiels comprises entre 1 mV.s⁻¹ et 10 000 mV.s⁻¹, de préférence comprises entre 10 mV.s⁻¹ et 1 000 mV.s⁻¹, plus préférablement comprises entre 10 mV.s⁻¹ et 100 mV.s⁻¹, sans mesure de la variation du courant, permettant l'oxydation puis la réduction de la partie de la surface active en or de l'électrode de mesure en contact avec le liquide alimentaire.

Il est également décrit un dispositif électrochimique (non selon l'invention) de détection et/ou quantification du SO₂ libre dans un liquide alimentaire aqueux ou hydroalcoolique caractérisé en ce qu'il comprend :
- une électrode de référence,
- une contre-électrode,
- une électrode de mesure dont la surface active est entièrement en or,
et caractérisé en ce que l'électrode de mesure a un rapport R_{théo} entre son aire développée (A_{dev}) et son aire apparente (Aₐₚₚ) supérieur ou égal à 3, de préférence compris entre 3 et 100, plus préférablement compris entre 3 et 20.

L'invention propose un dispositif électrochimique de détection et/ou quantification du SO₂ libre dans un liquide alimentaire aqueux ou hydroalcoolique, ledit dispositif étant configuré pour mettre en œuvre les étapes de la méthode selon l'invention, ledit dispositif étant caractérisé en ce qu'il comprend :
- une électrode de référence
- une contre-électrode
- une électrode de mesure dont la surface active est entièrement en or, cette électrode de mesure ayant un rapport R_{théo} entre son aire développée (A_{dev}) et son aire apparente (Aₐₚₚ) supérieur ou égal à 3, de préférence compris entre 3 et 100, plus préférablement compris entre 3 et 20, et
- un dispositif permettant la mise en œuvre d'une voltampérométrie cyclique avec un balayage en potentiel compris entre -1 V et +2 V, en utilisant une électrode de référence Ag/AgCl, à des vitesses de balayage en potentiel comprises entre 1 mV.s⁻¹ et 10000 mV.s⁻¹, de préférence entre 10 mV.s⁻¹ et 1000 mV.s⁻¹, plus préférablement comprises entre 10 mV.s⁻¹ et 100 mV.s⁻¹.

Selon un mode de réalisation du dispositif de l'invention, l'électrode de mesure est une électrode comprenant un support recouvert d'une gaine poreuse en or, cette gaine ayant une épaisseur et des tailles de pores telles que l'électrode de mesure a un rapport R_{théo} entre son aire développée (A_{dev}) et son aire apparente (Aₐₚₚ) supérieur ou égal à 3, de préférence compris entre 3 et 100, plus préférablement compris entre 3 et 20. Cette gaine poreuse forme la surface active de l'électrode de mesure.

La taille des pores et l'épaisseur de la gaine poreuse sont mesurées par microscopie électronique à balayage (MEB).

Selon un autre mode de réalisation du dispositif de l'invention, l'électrode de mesure est une électrode comprenant un support recouvert d'une couche rugueuse en or, constituée de cristallites en or de dimensions nano à micrométriques de sorte que l'électrode de mesure a un rapport R_{théo} entre son aire développée (A_{dev}) et son aire apparente (Aₐₚₚ) supérieur ou égal à 3, de préférence compris entre 3 et 100, plus préférablement compris entre 3 et 20. Cette couche rugueuse en or forme la surface active de l'électrode de mesure.

L'invention sera mieux comprise et d'autres caractéristiques et avantages de celle-ci apparaîtront plus clairement à la lecture de la description explicative qui suit et qui est faite en référence aux figures dans lesquelles :
- la figure 1 est un schéma d'un dispositif selon l'invention,
- la figure 2 représente une première électrode de mesure, dite « disque plan », utilisée dans la détection et/ou quantification de l'invention,
- la figure 3 montre une seconde électrode de mesure cylindrique, dite « microstructurée poreuse », en or utilisée dans le dispositif et la méthode de détection et/ou de quantification de l'invention,
- la figure 4 représente une photographie prise au microscope électronique à balayage (MEB) à un grandissement de 1000 de la surface de l'électrode représentée en figure 3,
- les figures 5a et 5b montrent des images prises au MEB à un grandissement de 1 000 de la surface d'une électrode de mesure cylindrique dite « microstructurée rugueuse » dont la surface est recouverte de cristallites d'or ;
- la figure 6 montre les courbes de voltampérométrie cyclique de solutions hydroalcooliques modèles avec ajouts de 0, 25 et 125 mg.L⁻¹ de SO₂ libre (respectivement courbe en pointillés, courbe en tirets et courbe pleine), obtenues en utilisant, comme électrode de mesure :
   - une électrode de mesure constituée d'un cylindre en or de 25 mm de longueur et de 250 µm de diamètre (figure 6A),
   - l'électrode microstructurée poreuse montrée en figure 3 et 4 (figure 6B),
   - l'électrode microstructurée rugueuse représentée en figure 5 (figure 6B),
   - l'électrode disque plan montrée en figure 2 (figure 6C), et
   - une électrode de mesure en or, d'un dispositif commercialisé par la société Dropsens qui comprend, en outre, une électrode de référence en Ag, et une contre-électrode, toutes ces électrodes étant déposées par sérigraphie dans un support en un matériau polymère (figure 6D),

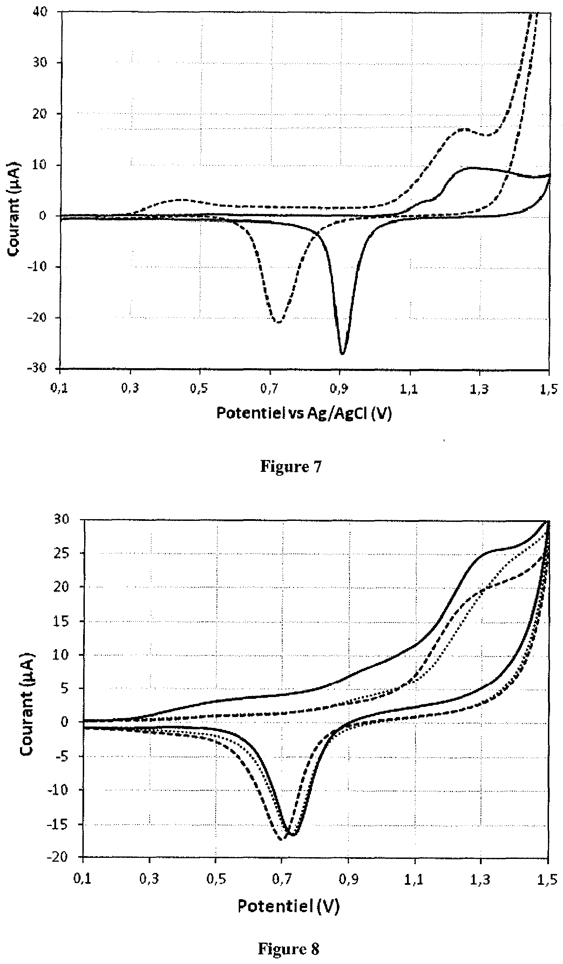

- la figure 7 montre les courbes de voltampérométrie cyclique obtenues dans une solution d'acide sulfurique 0,1 M (courbe en trait plein) et dans une solution hydroalcoolique modèle contenant 25 mg.L⁻¹ de SO₂ libre (courbe en tirets),
- la figure 8 montre les courbes de voltampérométrie cyclique obtenues avec une électrode de mesure en or du type disque plan dans un vin blanc (courbe pleine), un vin rosé (courbe en pointillés) et un vin rouge (courbe en tirets),

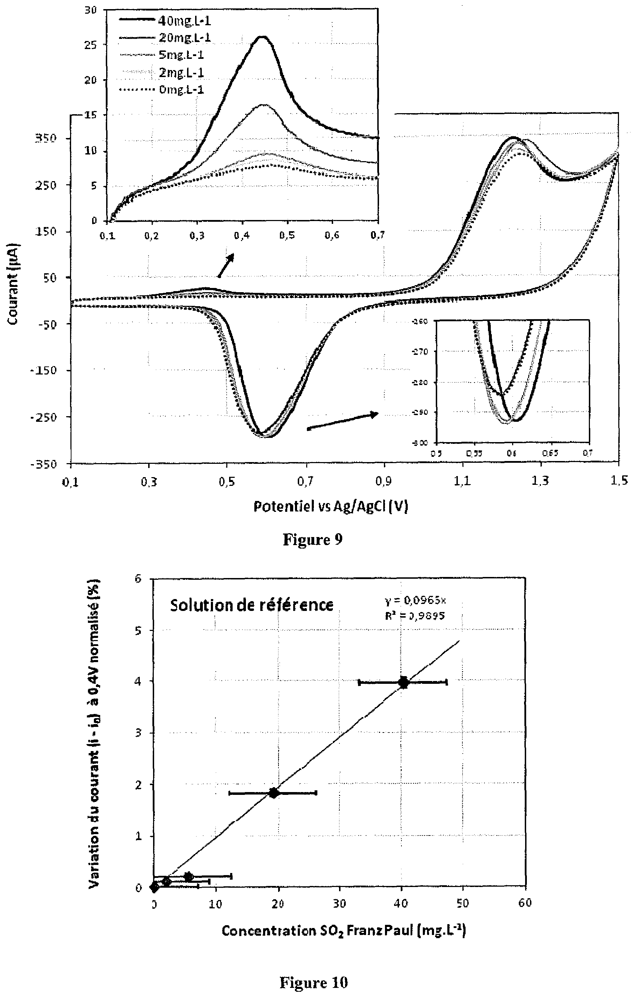

- la figure 9 montre les courbes de voltampérométrie cyclique enregistrées dans des solutions hydroalcooliques modèles contenant des teneurs variables en SO₂ libre en utilisant comme électrode de mesure une électrode microstructurée poreuse en or,
- la figure 10 montre la courbe de régression de la variation du courant normalisé mesuré à +0,4 V en fonction de la concentration en SO₂ libre dans une solution hydroalcoolique modèle,

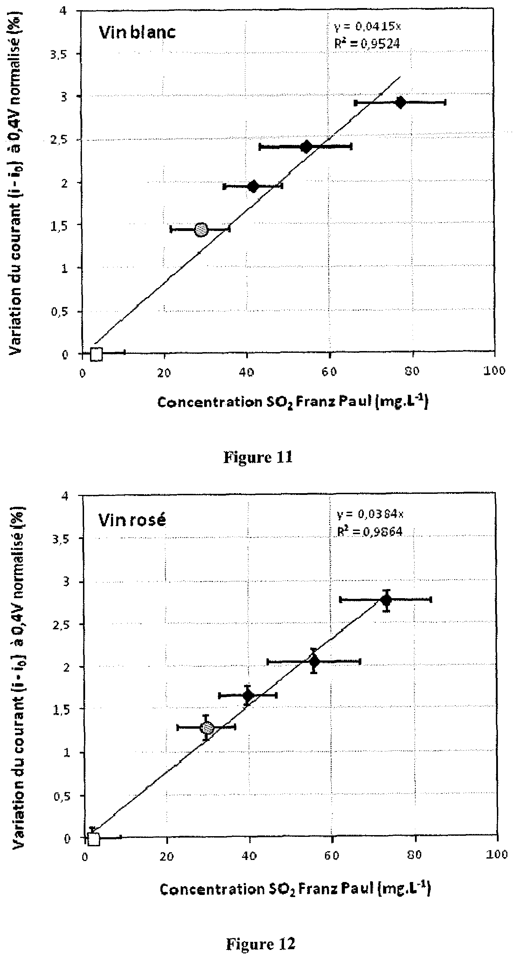

- la figure 11 montre les courbes de régression de la variation du courant normalisé mesuré à +0,4 V en fonction de la concentration en SO₂ mesurée par la méthode Franz Paul dans :
- un vin blanc (Bordeaux, Sauvignon Roche Mazet, 2014) (symbole rond gris), pour le même vin blanc auquel a été ajouté de l'éthanal (symbole carré blanc), et
- le même vin blanc avec des ajouts dosés de SO₂ de 20 mg.L⁻¹, 40 mg.L⁻¹ et 70 mg.L⁻¹ (symboles losanges noirs),
- la figure 12 montre les courbes de régression de la variation du courant normalisé mesuré à +0,4 V en fonction de la concentration en SO₂ mesurée par la méthode Franz Paul pour :
- un vin rosé (Cabernet d'Anjou, Plessis Duval, 2015) (symbole rond gris),
- le même vin rosé auquel a été ajouté de l'éthanal (symbole carré blanc), et
- le même vin rosé avec des ajouts dosés de SO₂ de 20 mg.L⁻¹, 40 mg.L⁻¹ et 70 mg.L⁻¹ (symboles losanges noirs),

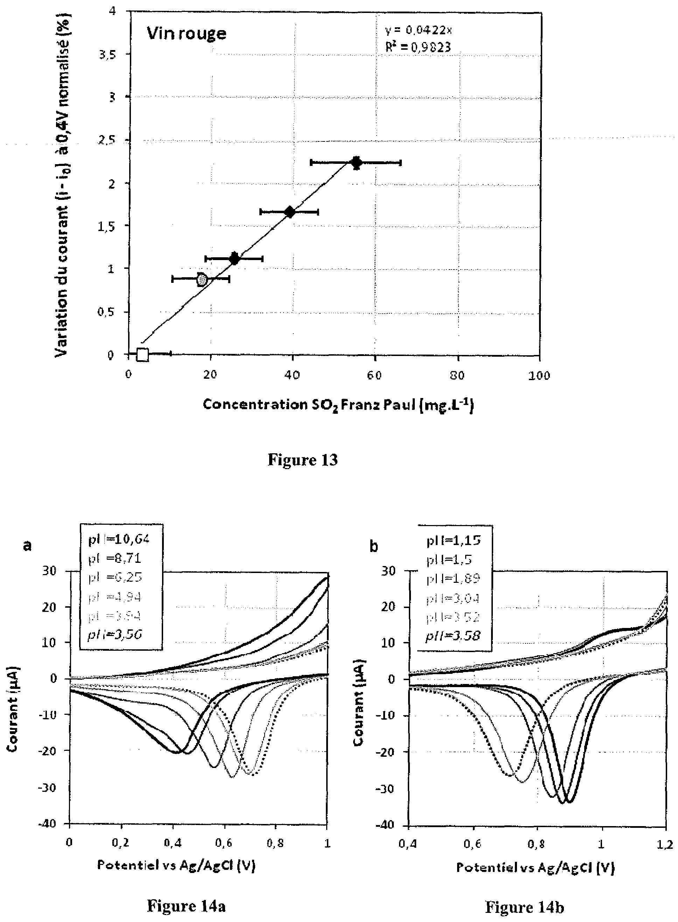

- la figure 13 montre les courbes de régression de la variation du courant normalisé mesuré à +0,4 V en fonction de la concentration en SO₂ mesurée par la méthode Franz Paul pour :
- un vin rouge (Morgon, Club des sommeliers, 2014) (symbole rond gris),
- le même vin rouge auquel a été ajouté de l'éthanal (symbole carré blanc), et
- le même vin rouge avec des ajouts dosés de SO₂ de 20 mg.L⁻¹, 40 mg.L⁻¹ et 70 mg.L⁻¹ (symboles losanges noirs),

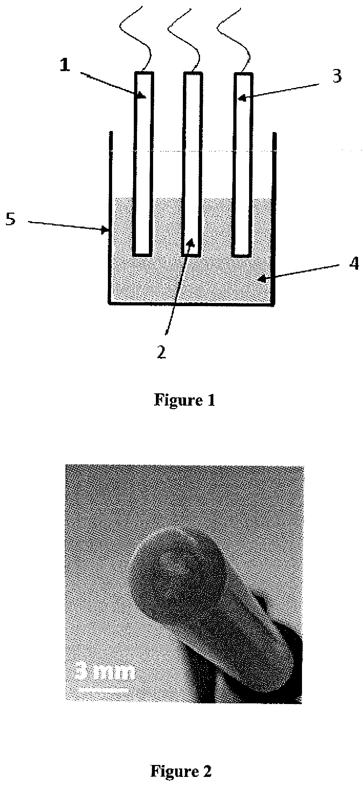

- la figure 14a montre les courbes de voltampérométrie cyclique, obtenues en utilisant comme électrode de mesure l'électrode disque plan montrée en figure 2, et enregistrées dans un vin blanc (courbe en pointillés) et en faisant varier le pH par ajout de soude (courbes en trait plein),
- la figure 14b montre les courbes de voltampérométrie cyclique, obtenues en utilisant, comme électrode de mesure, l'électrode disque plan montrée en figure 2, et enregistrées dans un vin blanc (courbe en pointillés) et en faisant varier le pH par ajout d'acide sulfurique (courbes en trait plein), et

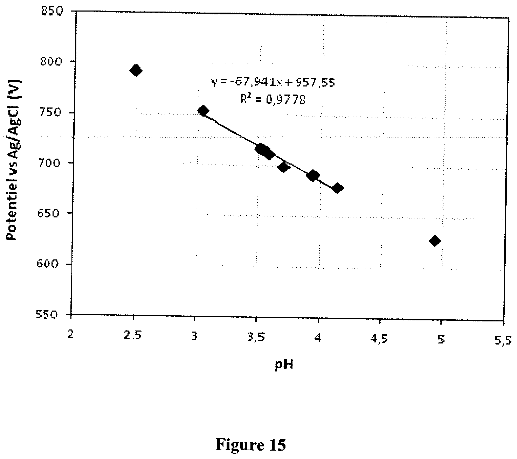

- la figure 15 montre la variation de la position du pic de réduction des oxydes d'or en fonction du pH pour un vin blanc lorsqu'une électrode de mesure qui est l'électrode disque plan montrée en figure 2 est utilisée.

L'invention propose une méthode de détection et/ou quantification du SO₂ libre dans un liquide alimentaire aqueux ou hydroalcoolique basée sur une mesure électrochimique par voltampérométrie cyclique dans laquelle une électrode, dont la surface active est entièrement en or, est utilisée en tant qu'électrode de mesure.

Dans la présente invention les termes 'surface active' désignent la surface de la couche destinée à être connectée électriquement à un potentiostat et qui réagit avec les sulfites.

Dans la présente invention par 'surface active entièrement en or', on entend que la surface active est constituée uniquement d'or d'une pureté supérieure à 95%, de préférence supérieure à 99%

Dans la présente invention par 'liquide alimentaire aqueux' ou 'hydroalcoolique', on entend, pour les liquides aqueux, les jus de fruits en particulier et pour les liquides hydroalcooliques, on entend le vin effervescent ou non, le champagne, le cidre, les alcools à base de fruits, et la bière.

La quantification et la détection du SO₂ libre sont effectuées dans une cellule électrochimique à trois électrodes schématisée en figure 1. Elle comporte trois électrodes : une électrode de mesure, notée 1 en figure 1, une électrode de référence, notée 2 en figure 1, et une contre-électrode, notée 3 en figure 1, qui sont plongées dans un liquide alimentaire, noté 4 en figure 1, contenu dans un récipient, noté 5 en figure 1, dont on veut connaître la teneur en SO₂ libre. Ces trois électrodes sont connectées à un potentiostat (non montré) qui peut transmettre les données mesurées à un ordinateur.

L'électrode de mesure 1 peut être une électrode de mesure de type disque plan, montrée en figure 2, qui est constituée par la section droite d'un fil d'or, par exemple de 3 mm de diamètre, enrobé par un corps isolant, par exemple en Téflon^{®}.

L'électrode de mesure 1 peut encore être un cylindre en or, par exemple d'une longueur de 25 mm et un diamètre de 250 µm.

L'électrode de mesure 1 peut aussi être une électrode de mesure en or déposée par sérigraphie sur un support en polymère où, outre l'électrode de mesure en or, une électrode de référence en argent et une contre-électrode en or sont également déposées par sérigraphie sur le même support en polymère.

Cet ensemble constitue un système jetable qui pourra alors être directement plongé dans l'échantillon pour la mesure et relié à un potentiostat.

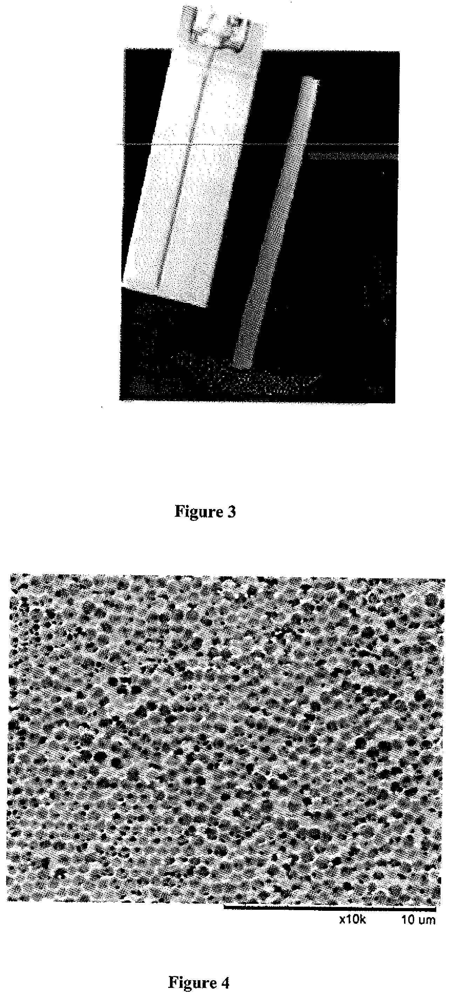

Mais, avantageusement, l'électrode de mesure 1 sera une électrode microstructurée poreuse, telle représentée en figure 3, qui consiste en un support cylindrique dont au moins la surface externe est en or, d'environ 25 mm de longueur et 250 µm de diamètre, ladite surface externe étant revêtue d'une gaine poreuse en or permettant d'accroître sa surface active. Cette gaine poreuse forme la surface active de l'électrode de mesure. Selon une forme de réalisation, le support cylindrique peut être intégralement constitué d'or. Cependant, le cylindre peut être formé d'un cœur en verre ou en métal autre que l'or et recouvert d'une couche d'or. Cette couche d'or forme la surface active de l'électrode de mesure. Bien que le support montré en figure 3 soit cylindrique, il apparaitra clairement à l'homme de l'art que le support peut être un support plan, ou encore hémisphérique.

Des électrodes microstructurées poreuses, telles que celle représentée en figure 3 peuvent être obtenues comme décrit dans la demande internationale WO 2016/030806 : des couches de particules sphériques de silice de tailles contrôlées sont déposées sur la surface du cylindre. Le diamètre des particules de silice déposées peut varier de 50 nm jusqu'à 5 µm. Puis, une électrodéposition d'or à travers les interstices du film de particules de silice est effectué. Ensuite, les particules de silice sont éliminées par un traitement chimique, ce qui permet l'élaboration d'une gaine ayant une structure périodique poreuse en or, la taille des pores étant ajustée par le diamètre des particules. L'épaisseur de cette gaine poreuse est contrôlable entre l'équivalent de la mi-hauteur d'une couche de particules jusqu'à la hauteur correspondant à 50 couches de particules, soit une épaisseur de 25 nm à plusieurs centaines de µm. Une telle électrode développe ainsi une grande surface spécifique correspondant à la surface active de l'électrode de mesure tout en conservant une faible dimension. Une telle électrode microstructurée poreuse est montrée en figure 3. La figure 4 montre la surface externe de cette électrode correspondant la surface active de l'électrode de mesure.

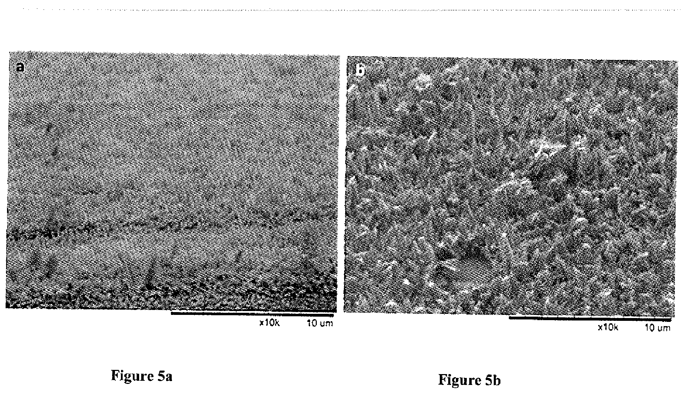

L'électrode de mesure peut aussi être une électrode microstructurée rugueuse, c'est à dire comportant une surface en or rugueuse constituée d'un réseau de cristallites en or, de dimension nano à micrométrique (de 100µm à 1 nm). Cette surface en or rugueuse forme la surface active de l'électrode de mesure. Une telle électrode est montrée en figures 5a et 5b. La surface microstructurée rugueuse a été obtenue par électrodéposition d'un sel d'or (acide tétrachloraurique) en présence d'acétate de plomb, comme décrit dans Plowman B. J., Ippolito S. J., Bansal V., Sabri Y. M., O'Mullane A. P., Bhargava S. K. Chem.Commun., 2009, 33, 5039, sur un support qui peut être cylindrique, plan ou sphérique, en or ou en un matériau différent de l'or mais recouvert d'une couche d'or. La durée de l'électrodéposition permet de contrôler la taille des aiguilles d'or et donc le rapport R_{théo} de l'électrode obtenue. Ainsi l'électrode en or microstructurée rugueuse montrée en figure 5a, pour laquelle la durée de l'électrodéposition était de 300 s, présente un rapport R_{théo} de 4,9 et l'électrode en or microstructurée rugueuse montrée en figure 5b, pour laquelle la durée de l'électrodéposition était de 600s, est caractérisée par un rapport R_{théo} de 12,6.

On a constaté que lorsque la surface spécifique de l'électrode de mesure en or était augmentée elle présentait une plus grande sensibilité de détection et/ou quantification du SO₂ libre tout en conservant une faible dimension. C'est pourquoi, dans le dispositif de détection électrochimique de l'invention, l'électrode de mesure en or présentant une surface active poreuse structurée (l'électrode microstructurée poreuse) est préférée.

Par surface poreuse structurée ou microstructurée, on entend que le rapport R_{théo} entre l'aire développée et l'aire apparente est supérieur ou égal à 3. De préférence, ce rapport est compris entre 3 et 100, et plus particulièrement entre 3 et 20.

L'aire apparente, notée Aₐₚₚ, est définie comme l'aire géométrique de l'électrode, c'est-à-dire que l'on multiplie simplement, pour une électrode plane, la longueur par la largeur de la surface de cette électrode.

L'aire développée, notée A_{dev}, est définie comme l'aire maximale exposée pouvant interagir avec la solution environnante. Elle correspond à l'aire réelle et reflète toutes les structurations possibles de la surface du matériau (porosité, rugosité, etc...).

Ce rapport R_{théo} est d'environ 1 pour l'électrode disque plan, et pour l'électrode en or cylindrique nue (sans microstructuration rugueuse ou poreuse), utilisées pour détecter et/ou quantifier le SO₂ libre dans l'invention.

Pour l'électrode microstructurée poreuse dont la gaine poreuse est constituée de sept couches et demi d'or poreuses avec des pores de 1170 nm de diamètre moyen mesuré par MEB, ce rapport R_{théo} est d'environ 11,8 et pour l'électrode microstructurée poreuse, dont la gaine poreuse est constituée de deux couches et demie d'or ayant des pores de 585 nm de diamètre moyen mesuré par MEB, ce rapport R_{théo} est d'environ 3,9.

L'électrode de mesure 1 peut être une électrode microstructurée poreuse obtenue par dépôt par un procédé d'impression d'une couche d'or microstucturée poreuse sur un support dont la surface peut être, par exemple en carbone, platine, argent ou or.

L'électrode de mesure 1 peut également comprendre une membrane protectrice ou sélective entourant la surface active entièrement en or.
Cette membrane peut être une membrane d'exclusion de taille ou de charge (anionique ou cationique) déposée par différentes méthodes telles que la coulée de goutte (drop-casting en anglais), le trempage (dip-coating en anglais), le dépôt laminaire ou l'électrophorèse.

La méthode de l'invention permet de quantifier très précisément le SO₂ libre présent dans un liquide alimentaire grâce à une courbe de calibration préalablement générée.

En effet, l'électrode en or présente une excellente sélectivité au SO₂ libre et il est possible de régénérer sa surface active directement dans le liquide alimentaire aqueux ou hydroalcoolique, ce qui permet ainsi de prolonger sa durée de vie tout en améliorant la répétabilité des mesures effectuées.

Dans la présente invention, on utilise la voltampérométrie cyclique qui consiste à appliquer un balayage en potentiel linéaire entre un potentiel initial et un potentiel final, et à mesurer les variations de courant résultant du transfert d'électrons générés par les processus d'oxydation ou de réduction produits au cours du cycle.

Dans l'invention elle consiste à appliquer des potentiels d'oxydation, puis de réduction en retour.

Ceci offre l'avantage de réduire des espèces créées au cours de l'oxydation et donc de revenir au potentiel de départ, et donc à l'état initial de l'électrode de mesure, à la fin de chaque cycle.

Cette méthode présente ainsi deux avantages techniques par rapport à d'autres méthodes mono-variantes telle que la voltamétrie linéaire :
- après plusieurs cycles de mesure, il est possible d'obtenir une réponse électrochimique stable de l'électrode qui permet de négliger les variations de l'état de surface initial de l'électrode ; et
- la mesure, au cours d'un cycle, d'un pic de courant caractéristique de la réduction des oxydes d'Or permet de normaliser les courants par un calcul précis de la surface électrochimiquement active et ainsi de s'affranchir de différences de comportement liées à la nature de la matrice utilisée ;

Selon la présente invention, le balayage en potentiel lors de la voltampéromètrie cyclique peut être effectué entre -1 V et +2 V, mais est de préférence effectué entre +0,1 V et +1,9 V, et plus préférablement entre + 0,1 V et +1,5 V. Ces valeurs de potentiels sont celles appliquées en utilisant une électrode de référence Ag/AgCl, à des vitesses de balayage en potentiels comprises entre 1 mV.s⁻¹ et 10 000 mV.s⁻¹, de préférence comprises entre 10 mV.s⁻¹ et 1 000 mV.s⁻¹, plus préférablement comprises entre 10 mV.s⁻¹ et 100 mV.s⁻¹.

Cependant, selon une forme de réalisation avantageuse de la méthode de l'invention, la gamme de potentiels est définie de manière à pouvoir, d'une part, mesurer le pic d'oxydation du SO₂ libre et, comme on le verra dans les exemples, d'autre part, régénérer l'électrode de mesure pendant le cycle de mesure. Ainsi de préférence, on applique un potentiel allant de +0,1 V à +1,5 V en utilisant une électrode de référence Ag/AgCl, à des vitesses de balayage en potentiel comprises entre 1 mV.s⁻¹ et 10 000 mV.s⁻¹, de préférence comprises entre 10 mV.s⁻¹ et 1 000 mV.s⁻¹, plus préférablement comprises entre 10 mV.s⁻¹ et 100 mV.s⁻¹.

La variation du courant produit par la réaction d'oxydation du SO₂ libre est alors mesurée, sauf dans le cas de la régénération de l'électrode.

L'invention propose encore un dispositif électrochimique de détection et/ou de quantification du SO₂ libre dans un liquide alimentaire aqueux ou hydroalcoolique comprenant une électrode de référence 2, de préférence en Ag/AgCl pour fixer le potentiel, une contre-électrode 3, de préférence en un polymère conducteur, tel du polycarbonate chargé avec des particules de carbone, du poly(pyrrole), et une électrode de mesure 1 microstructurée poreuse ou rugueuse, en or, telle que définie ci-dessus.

Cependant, on pourra également utiliser, en tant qu'électrode de référence 2, une électrode normale à hydrogène ou une électrode au calomel saturé par KCl ou NaCl.

De la même façon, une contre-électrode 3 en métal noble (platine), en acier inoxydable, ou en carbone non dégradable pourra également être utilisée.

La méthode de mesure par voltampérométrie cyclique est, comme cela sera démontré dans les exemples qui suivent, la méthode électrochimique à appliquer pour détecter et/ou quantifier de façon précise, sélective et répétable le SO₂ libre dans un liquide alimentaire aqueux ou hydroalcoolique.

Afin de mieux faire comprendre l'invention, ainsi que ces avantages, plusieurs modes de mise en œuvre, à titre d'exemples purement illustratifs et non limitatifs, vont maintenant être décrits.

Dans ces exemples, l'électrode de référence est une électrode Ag/AgCl, sauf lorsque l'électrode utilisée est l'électrode sérigraphiée, auquel cas l'électrode de référence est en Ag. La vitesse de balayage en potentiel est maintenue à 50 mV.s⁻¹.

### Exemple 1 : Comparaison des électrodes de mesure dans des solutions modèles

Des solutions modèles ayant une composition proche de celles des vins mais présentant une matrice plus simple ont été préparées.

La matrice de ces solutions modèles était constituée d'eau, d'éthanol et d'acide tartrique. La quantité d'éthanol était constante et égale à 12 % volumique, la concentration en acide tartrique fixée à 5g.L⁻¹ et le pH ajusté à 3,3 par ajout de soude à 30 %.

La quantité de SO₂ libre a été contrôlée dans une gamme de concentrations allant de 0 mg.L⁻¹ à 250 mg.L⁻¹ en ajoutant une quantité variable d'une solution d'anhydride sulfureux (SO₂) dont le titre est quantifié par la méthode de Franz Paul.

Dans ces solutions modèles, la totalité du SO₂ ajouté reste sous la forme de SO₂ libre en solution car elles ne contiennent pas de composés pouvant combiner une partie du SO₂ ajouté.

Dans cet exemple, les réponses électrochimiques de solutions modèles contenant une quantité croissante de SO₂ libre ont été étudiées en utilisant différentes électrodes de mesure. Pour chaque forme d'électrode de mesure, les mesures sont effectuées avec une quantité croissante de SO₂ libre. Ces quantités sont respectivement 0, 25 et 125 mg.L⁻¹ de SO₂ libre.

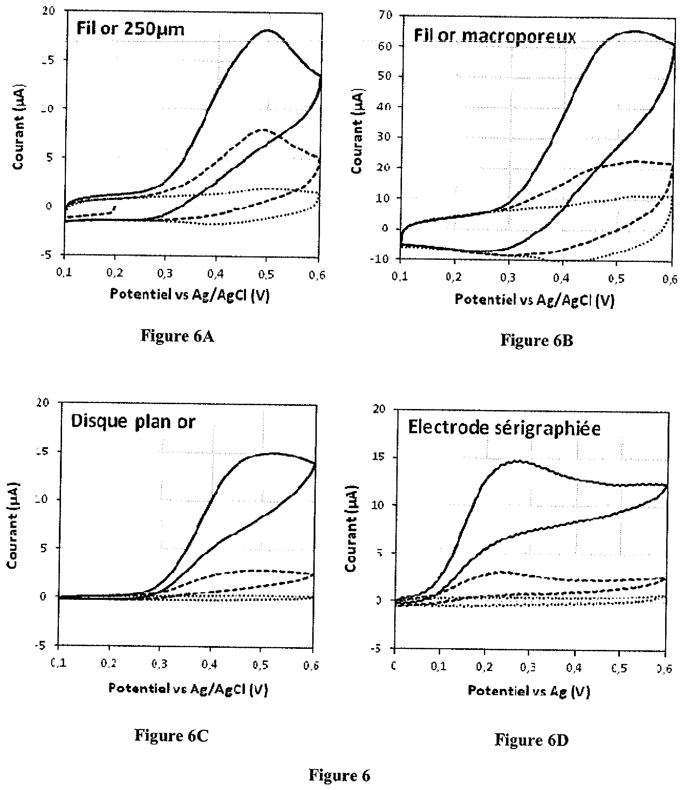

Ainsi, ont été testées :
- une électrode en or constituée d'un fil d'or nu d'une longueur de 20 mm et d'un diamètre de 250 µm ayant une aire apparente de 15,6 mm² et un R_{théo} de 0,8 : figure 6A,
- une électrode en or microstructurée poreuse constituée d'un fil en or recouvert d'une gaine poreuse en or ayant une aire apparente de 15,6 mm² et un R_{théo} = 11,4 : figure 6B,
- une électrode en or disque plan avec un diamètre de 3 mm ayant une aire apparente de 7 mm² et un R_{théo} de 1,3 : figure 6C, et
- une électrode en or sérigraphiée ayant une aire apparente de 12 mm² et un R_{théo} de 1 : Figure 6D.

Tous ces voltampérogrammes montrent une augmentation du courant lorsque la concentration en SO₂ libre augmente (0, 25 et 125 mg.L⁻¹), à partir de +0,1 V (vs Ag/AgCl) pour l'électrode sérigraphiée et à partir de +0,3 V (vs Ag/AgCl pour les trois autres types d'électrodes de mesure utilisées. Ceci traduit la spécificité de la réponse électrochimique au SO₂ libre de l'électrode de mesure en or, cette espèce étant la seule dont la concentration varie.

Les augmentations du courant enregistrées correspondent à l'oxydation du SO₂ libre. On constate que l'intensité des courants mesurés est plus élevée lorsque l'électrode de mesure est une électrode en or microstructurée poreuse. Cette électrode est donc préférée car présentant une grande sensibilité.

L'électrode en or microstructurée poreuse testée est ici une électrode dont la gaine externe a une épaisseur de 15 µm et des pores d'un diamètre moyen de 1170 nm mesuré par MEB. Cette gaine externe forme la surface active de l'électrode de mesure.

Les trois autres électrodes présentent des intensités de courant mesurées comparables, malgré leurs aires apparentes différentes, mais en accord avec leur valeur du rapport R_{théo} proche de 1.

On peut également constater à partir de la figure 6, que la position du maximum de courant est décalée vers +0,25 V dans le cas de l'électrode sérigraphiée, car l'électrode de référence (ici en Ag) est différente de celle des trois autres systèmes (électrode de référence Ag/AgCl).

### Exemple 2 : Répétabilité des mesures et régénération de l'électrode de mesure

La répétabilité de la réponse électrochimique de solutions hydroalcooliques modèles contenant 125 mg.L⁻¹ de SO₂ libre a été étudiée en utilisant l'électrode disque plan en or sans nettoyage électrochimique préalable de celle-ci.

Les voltampérogrammes obtenus montrent une très grande disparité des réponses, tant au niveau de l'intensité que de la position du maximum de la vague d'oxydation du SO₂ libre. Ceci révèle que l'état de surface de l'électrode n'est pas le même avant chaque expérience en raison de l'adsorption de molécules à la surface de l'électrode.

La régénération de la surface de l'électrode, avant chaque mesure est donc nécessaire.

Un protocole standard de régénération de l'électrode de mesure utilisé dans les laboratoires consiste à réaliser des cycles dans une large gamme de potentiels (entre +0,1 V et +1,5 V) dans une solution d'acide sulfurique (H₂SO₄) de concentration comprise entre 0,1 et 0,5 M jusqu'à l'obtention d'un signal stable.

Le voltampérogramme cyclique obtenu avec l'électrode disque plan en or est caractéristique d'une électrode en or avec l'apparition d'une vague à +1,3 V qui correspond à l'oxydation de la surface d'or et un pic intense à +0,9 V dans la branche cathodique due à la réduction des oxydes d'or précédemment générés.

Les solutions hydroalcooliques modèles ayant un pH de 3,3 ont été utilisées pour confirmer ou infirmer que les électrodes de mesure utilisées dans le dispositif et la méthode de l'invention pouvaient être régénérées directement dans le milieu contenant le SO₂ libre, l'acidité de celui-ci étant donc suffisante.

Ainsi des cycles dans une gamme de potentiels entre +0,1 V et +1,5V dans la solution modèle contenant 25 mg.L⁻¹ du SO₂ libre ont été réalisés.

Les courbes de voltampérométrie cyclique obtenues sont montrées en figure 7. Elles présentent la vague d'oxydation du SO₂ libre présente à +0,45 V, et également le pic premier d'oxydation de l'or vers +1,25 V et le pic bien défini de réduction des oxydes d'or à +0,75 V.

La couche d'oxydes créée pendant le régime anodique peut conduire à une décroissance du courant mesuré et une perte de sensibilité progressive de l'électrode.

La réduction électrochimique des oxydes d'or au cours du régime cathodique permet alors d'éliminer cette couche inhibitrice, en effectuant un nettoyage et une réactivation de la surface de l'électrode.

Ainsi, il a été démontré qu'il est possible de régénérer l'électrode de mesure directement dans le milieu de mesure, c'est-à-dire dans le liquide alimentaire aqueux ou hydroalcoolique, ce qui est un avantage certain.

Un tel procédé de régénération, au cours duquel la variation de l'intensité du courant n'est pas mesurée, mais seule l'obtention d'un signal stable est observée, est un objet de l'invention.

### Exemple 3 : Détermination de la fenêtre de potentiel de balayage lors de la voltampérométrie cyclique

Pour optimiser la fenêtre de potentiels à balayer pour la voltampérométrie cyclique dans la méthode de l'invention, on a fait varier la fenêtre de potentiel en gardant la borne inférieure égale à +0,1 V, c'est-à-dire au pied de la vague d'oxydation du SO₂ libre et en balayant au-delà du premier pic d'oxydation de l'or (vers +1,2 V) vers des potentiels plus positifs de +1,5 V à +1,9 V.

A partir de +1,4 V, le signal augmente fortement en raison de l'oxydation de l'eau en dioxygène. Il n'y a pas d'influence de la limite haute de potentiel sur le pic d'oxydation du SO₂ libre.

Cependant, pour diminuer le temps de mesure, la limite supérieure de la fenêtre de potentiel est de préférence de +1,5 V.

La fenêtre de potentiel a également été modifiée en jouant sur la limite inférieure de potentiel, de -0,4 V à +0,1 V.

Une vague apparaît dans le régime cathodique vers -0,2 V ce qui correspond à la réduction du SO₂ libre en soufre.

Du fait de sa forte affinité avec l'or, le soufre ainsi formé s'adsorbe sur la surface de l'électrode de mesure. La réponse de l'électrode au SO₂ libre est ainsi modifiée.

De plus, on constate un léger décalage du pic d'oxydation des sulfites vers les potentiels plus faibles et une diminution de l'intensité du pic.

D'autre part, le tracé de la variation du courant d'oxydation mesurée au potentiel de +0,4 V en fonction de la concentration en SO₂ libre présente un coefficient de régression très proche de 1 lorsque la mesure par voltampérométrie cyclique est effectuée entre +0,1 V et +1,5 V alors que la linéarité de la réponse aux faibles concentrations de SO₂ libre (≤ 15 mg.L⁻¹) est perdue pour la mesure de voltampérométrie cyclique effectuée entre -0,4 V et +1,5 V.

Ainsi, dans la méthode de l'invention, la mesure de voltampérométrie cyclique doit être effectuée entre +0,1 V et +1,9 V, plus préférablement entre +0,1 V et +1,5 V, cette fenêtre permettant en effet de régénérer l'électrode tout en ayant une réponse spécifique au SO₂ libre.

Pour confirmer ce point, la figure 8 montre les voltampérogrammes cycliques enregistrés en utilisant une électrode de mesure disque plan dans un vin rouge, un vin rosé et un vin blanc entre des potentiels de +0,1 V et +1,5 V.

Ce voltampérogramme présente le pic d'oxydation de l'or aux alentours de +1,25 V et le pic bien symétrique de la réduction des oxydes d'or vers +0,75 V.

La surface de l'électrode de mesure peut donc être directement régénérée dans les différents vins, sans réaliser cette opération de régénération dans une solution d'acide sulfurique.

Des mesures de répétabilité ont été menées en enregistrant la réponse électrochimique de six échantillons par vin.

Toutes les courbes de voltampérométrie présentent une inflexion aux alentours de +0,5V correspondant au pic de courant d'oxydation du SO₂ libre sur électrode d'or.

Pour tous les vins, on observe une bonne répétabilité dans la pente du pic de l'oxydation du SO₂ libre (entre +0,1 V et +0,4 V), l'écart entre les mesures étant plus significatif au-delà de +0,4 V.

Les écarts-types en % obtenus sur les courants d'oxydation mesurés à +0,4 V sont satisfaisants pour trois types de vins analysés.

| | Blanc | Rosé | Rouge |
|---|---|---|---|
| Courant à +0,4 V (µA) | 1,46 ± 0,2 (14%) | 0,80 ± 0,04 (5%) | 0,57 ± 0,05 (9%) |

Le courant d'oxydation est plus important pour le vin blanc et du même ordre de grandeur pour les rosé et rouge, ce qui tend à montrer que la concentration en SO₂ libre est plus importante dans le vin blanc que dans les deux autres vins.

Ceci est confirmé par le dosage du SO₂ libre contenu dans ces vins effectué par un dispositif utilisant la méthode Ripper.

| | Blanc | Rosé | Rouge |
|---|---|---|---|
| SO₂ libre Ripper (mg.L⁻¹) | 55 ± 11 (20%) | 11 ± 7 (63%) | 14 ± 7 (50%) |

### Exemple 4. Etablissement d'une courbe de calibration normalisée dans la solution modèle

Dans cet exemple, des voltampérogrammes cycliques ont été enregistrés entre +0,1 V et +1,5 V avec l'électrode de mesure en or microstructurée poreuse.

Cette électrode de mesure microstructurée poreuse est constituée d'un fil en or de 25 mm de longueur et 250 µm de diamètre présentant une couche poreuse en surface, dont l'épaisseur est de 2,7 µm et le diamètre moyen des pores, mesuré par MEB, est de 585 nm.

Les échantillons sont des solutions modèles hydroalcooliques contenant des quantités en SO₂ libre variables et contrôlées par ajout d'une quantité variable d'une solution de SO₂ dont le titre est quantifié par la méthode Franz Paul.

La figure 9 montre les courbes de voltampérométrie cyclique obtenues.

Comme pour l'électrode en or disque plan, une augmentation quantitative du courant avec la concentration en SO₂ libre est enregistrée à +0,2 V avec un maximum vers approximativement +0,45 V qui correspond à l'oxydation du SO₂ libre.

Le fait d'appliquer à l'échantillon un balayage linéaire dans une large gamme de potentiels (entre +0,1 V et +1,5 V) permet à la fois de mesurer le pic d'oxydation du SO₂ libre mais aussi de régénérer l'électrode de mesure microstructurée directement dans l'échantillon avec l'apparition d'une vague vers +1,2 V qui correspond à l'oxydation de la surface d'or de l'électrode et un pic intense aux alentours de +0,6 V dans la branche cathodique due à la réduction des oxydes d'or régénérés.

On constate d'après les voltampérogrammes reportés en figure 9, que l'électrode microstructurée poreuse présente une plus grande sensibilité pour le dosage du SO₂ libre, les courants variant entre -350 µA et +350 µA alors qu'avec l'électrode disque plan, ils variaient entre -50 µA et +50 µA.

Par ailleurs, la courbe de régression de la variation du courant mesuré à +0,4 V en fonction de la concentration en SO₂ libre dans une solution contenant de 0 à 40 mg.L⁻¹ de SO₂ ajouté a été tracée en figure 10.

Les variations des courants d'oxydation mesurés à +0,4 V sont normalisées par la hauteur du pic de la réduction des oxydes d'or observés dans chaque voltampérogramme cyclique de façon à tenir compte de la surface active réelle *in situ* de l'électrode au cours des mesures.

Comme on le voit en figure 10, lorsque la valeur du courant d'oxydation mesuré au potentiel de +0,4 V est tracée en fonction de la concentration en SO₂ libre mesurée par la méthode Franz Paul, on obtient un coefficient de régression très proche de 1, ce qui traduit une nouvelle fois que la réponse électrochimique enregistrée est bien spécifique au SO₂ libre qui est la seule espèce dont la concentration varie linéairement.

On observe encore, à partir de la figure 9, un décalage de la position du pic de réduction des oxydes d'or vers les potentiels plus positifs avec l'augmentation de la concentration en SO₂ libre.

La position en potentiel du pic de réduction des oxydes d'or étant dépendante du pH, son déplacement corrobore l'acidification de la solution par l'augmentation de la quantité de SO₂. Il est donc possible de relier la position de ce pic au pH de la solution, et d'établir une courbe d'étalonnage (abaque) qui permettra de déterminer le pH du liquide alimentaire à partir de la position du pic de réduction des oxydes d'or, dans ce liquide, lors d'une voltampérométrie cyclique.

Ainsi, le dispositif électrochimique et la méthode électrochimique de l'invention permettent également de mesurer le pH du liquide alimentaire sans avoir à ajouter une nouvelle électrode.

Ceci est un avantage supplémentaire du dispositif électrochimique et de la méthode électrochimique de l'invention.

### Exemple 5: Etablissement de courbes de calibration normalisées dans différents vins : vin blanc, vin rosé, et vin rouge

De la même façon, les courbes de régression de la variation du courant normalisé mesuré à +0,4 V en fonction de la concentration en SO₂ libre mesurée par la méthode Franz Paul ont été tracées dans un vin blanc, dans un vin rosé et dans un vin rouge (Figures 11, 12, 13 respectivement).

L'électrode de mesure utilisée est la même que celle utilisée à l'exemple 4 (électrode microstructurée poreuse constituée d'un fil en or de 25 mm de longueur et 250 µm de diamètre ayant une gaine poreuse, dont l'épaisseur est de 2,7 µm et le diamètre moyen des pores, mesuré par MEB, est de 585 nm).

Les courbes de régression ont été construites en mesurant la réponse électrochimique des vins (symbole rond et gris dans les Figures 11, 12, 13), des vins avec ajouts dosés de SO₂ (symboles losanges et noirs dans les Figures 11, 12, 13) et des vins avec ajout d'éthanal (symbole carré et blanc dans les Figures 11, 12, 13). L'ajout d'éthanal permet de combiner le SO₂ libre présent dans les vins et le voltampérogramme cyclique enregistré correspond en quelque sorte à la ligne de base, le signal du au SO₂ libre étant éliminé.

Ces courbes mettent en évidence une augmentation linéaire du courant en fonction de la concentration en SO₂ libre pour les trois types de vins. Les pentes de ces droites sont très proches mais sont en revanche inférieures à celle obtenue dans le cas de la solution modèle de l'exemple 4, ce qui traduit l'effet de la matrice vin sur la mesure de la concentration en SO₂ libre.

### Exemple 6 : Mesure du pH et de la concentration en SO₂ moléculaire

On a alors effectué une mesure du pH en suivant le protocole de mesure précédemment décrit, à partir de la position du pic de réduction des oxydes d'or précédemment décrit et avec un abaque préalablement réalisé reliant la position de ce pic à la valeur du pH.

La position du pic de réduction des oxydes d'or varie avec le pH (mesuré avec un pH-mètre) comme le montre la figure 14.

La mesure du pH peut donc être obtenue via l'électrode de mesure, sans avoir recours à une électrode à pH supplémentaire en solution.

Ainsi, il existe une relation linéaire entre la position du pic de réduction des oxydes d'or et le pH, notamment dans la gamme de pH des vins (pH 3 à 4), comme le montre la figure 15.

A partir de la mesure de la concentration en SO₂ libre obtenue, du pH, de la détermination, en parallèle de la température (T) de l'échantillon à l'aide d'une sonde classique, et du titre alcoolique (TAV) de l'échantillon, il est possible de remonter à la concentration en SO₂ moléculaire ou actif grâce aux deux formules suivantes (*cf* : Le SO2 en œnologie, Jacques Blouin, Dunod, 2014**)** : ${SO}_{2} actif \left(% {SO}_{2} libre\right) = 100 / \left[{10}^{\left(pH-pK1\right)}+1\right]$ avec ${pK}_{1} = 1 , 9499 + \left(T-20\right) \times 0 , 0322 + \left(TAV-10\right) \times 0 , 01971 .$

La température est mesurée sur l'échantillon juste avant, pendant ou après, la mise en œuvre de la méthode de dosage de l'invention. Le titre alcoolique est déterminé, soit par une mesure effectuée par la personne mettant en œuvre la méthode de l'invention juste avant, pendant ou après, la mise en œuvre de la méthode de dosage de l'invention, soit il est connu car il a été mesuré auparavant, par exemple par le vinificateur.

## Revendications

1. Méthode électrochimique de détection et/ou quantification du dioxyde de soufre (SO₂) sous ses formes non complexées dites libres dans un liquide alimentaire aqueux ou hydroalcoolique **caractérisé en ce qu'**elle comprend :
a) l'introduction dans le liquide alimentaire (4) d'une électrode de mesure (1) dont la surface active en contact avec le liquide alimentaire est entièrement en or, cette électrode de mesure ayant un rapport R_{théo} entre son aire développée (A_{dev}) et son aire apparente (Aₐₚₚ) supérieur ou égal à 3,
b) la mesure de la variation du courant produit par l'oxydation du SO₂ libre, présent dans le liquide alimentaire, lors d'un balayage en potentiel par voltampérométrie cyclique effectué entre -1 V et +2 V, en utilisant une électrode de référence Ag/AgCl (2), à des vitesses de balayage en potentiel comprises entre 1 mV.s⁻¹ et 10000 mV.s⁻¹, de préférence entre 10 mV.s⁻¹ et 1000 mV.s⁻¹, plus préférablement comprises entre 10 mV.s⁻¹ et 100 mV.s⁻¹ ladite méthode comprend avant l'étape b), une étape b1) de balayage en potentiel, lors d'une voltampérométrie cyclique, effectuée entre +0,1 V et +1,5 V, en utilisant une électrode de référence Ag/AgCl, à des vitesses de balayage en potentiel comprises entre 1 mV.s⁻¹ et 10000 mV.s⁻¹, de préférence entre 10 mV.s⁻¹ et 1000 mV.s⁻¹, préférablement comprises entre 10 mV.s⁻¹ et 100 mV.s⁻1, sans mesure de la variation du courant.

2. Méthode selon la revendication 1, **caractérisée en ce que**, à l'étape b), le balayage en potentiel par voltampérométrie cyclique est effectué entre +0,1 V et +1,9 V, de préférence entre +0,1 V et +1,5 V.

3. Méthode selon la revendication 1 ou 2, **caractérisée en ce que** le liquide alimentaire est du vin.

4. Méthode selon l'une quelconque des revendications précédentes, **caractérisée en ce que** l'électrode de mesure a un rapport R_{théo} compris entre 3 et 100, de préférence entre 3 et 20.

5. Méthode selon l'une quelconque des revendications précédentes, **caractérisée en ce qu'**elle comprend, de plus :
- avant l'étape b), une étape b2) d'établissement d'une abaque donnant la valeur du pH du liquide alimentaire en fonction de la position du pic de réduction des oxydes d'or lors de balayages en potentiel par voltampérométrie cyclique effectués dans ledit liquide alimentaire à différents pH, en utilisant une électrode de référence Ag/AgCl, à des vitesses de balayage en potentiel comprises entre 1 mV.s⁻¹ et 1000 mV.s⁻¹, de préférence entre 10 mV.s⁻¹ et 1000 mV.s⁻¹, préférablement comprises entre 10 mV.s⁻¹ et 100 mV.s⁻¹,
- la détermination du titre alcoolique, TAV, du liquide alimentaire, et
- pendant l'étape b) la mesure de la température T du liquide alimentaire, ce par quoi on obtient le pourcentage de SO₂ moléculaire contenu dans le SO₂ non complexé, grâce à la formule suivante : $% {SO}_{2} moléculaire = 100 / \left[{10}^{\left(pH-pK1\right)}+1\right]$ dans laquelle ${pK}_{1} = 1 , 949 + \left(T-20\right) \times 0 , 0322 + \left(TAV-10\right) \times 0 , 01971$

6. Dispositif électrochimique de détection et/ou quantification du SO₂ libre dans un liquide alimentaire aqueux ou hydroalcoolique, ledit dispositif étant configuré pour mettre en œuvre les étapes de la méthode selon l'une quelconque des revendications 1 à 5, ledit dispositif comprenant :
- une électrode de référence (2)
- une contre-électrode (3)
- une électrode de mesure (1) dont la surface active est entièrement en or, cette électrode de mesure ayant un rapport R_{théo} entre son aire développée (A_{dev}) et son aire apparente (Aₐₚₚ) supérieur ou égal à 3, de préférence compris entre 3 et 100, plus préférablement compris entre 3 et 20, et
- un dispositif permettant la mise en œuvre d'une voltampérométrie cyclique avec un balayage en potentiel compris entre -1 V et +2 V, en utilisant une électrode de référence Ag/AgCl, à des vitesses de balayage en potentiel comprises entre 1 mV.s⁻¹ et 10000 mV.s⁻¹, de préférence entre 10 mV.s⁻¹ et 1000 mV.s⁻¹, plus préférablement comprises entre 10 mV.s⁻¹ et 100 mV.s⁻¹.

7. Dispositif selon la revendication 6, **caractérisé en ce que** le dispositif permet la mise en œuvre d'une voltampérométrie cyclique avec un balayage en potentiel compris entre +0,1 V et +1,9 V, de préférence entre +0,1 V et +1,5 V.

8. Dispositif selon la revendication 6 ou 7, **caractérisé en ce que** l'électrode de mesure est une électrode comprenant un support recouvert d'une gaine poreuse en or, cette gaine ayant une épaisseur et une taille de pores telles que l'électrode de mesure a un rapport R_{théo} entre son aire développée (A_{dev}) et son aire apparente (Aₐₚₚ) supérieur ou égal à 3, de préférence compris entre 3 et 100, plus préférablement compris entre 3 et 20.

9. Dispositif selon la revendication 6 ou 7, **caractérisé en ce que** l'électrode de mesure est une électrode comprenant un support recouvert d'une couche rugueuse en or constituée d'un réseau de cristallites en or de dimensions nano à micrométriques de sorte que l'électrode de mesure a un rapport R_{théo} entre son aire développée (A_{dev}) et son aire apparente (Aₐₚₚ) supérieur ou égal à 3, de préférence compris entre 3 et 100, plus préférablement compris entre 3 et 20.

## Patentansprüche

1. Elektrochemisches Verfahren zum Nachweis und/oder zur Quantifizierung von Schwefeldioxid (SO₂) in seinen nicht komplexierten, sogenannten freien Formen in einer wässrigen oder hydroalkoholischen Nahrungsmittelflüssigkeit, **dadurch gekennzeichnet, dass** es umfasst:
a) das Einführen einer Messelektrode (1) in die Nahrungsmittelflüssigkeit (4), deren aktive Oberfläche, die mit der Nahrungsmittelflüssigkeit in Kontakt steht, vollständig aus Gold ist, wobei diese Messelektrode ein Verhältnis R_{théo} zwischen ihrer Abwicklungsfläche (A_{dev}) und ihrer Ansichtsfläche (Aₐₚₚ) größer oder gleich 3 aufweist,
b) das Messen der Änderung des durch die Oxidation des in der Nahrungsmittelflüssigkeit vorhandenen freien SO₂ erzeugten Stroms bei einer Potenzialabtastung durch zyklische Voltamperometrie zwischen -1 V und +2 V unter Verwendung einer Ag/AgCl-Referenzelektrode (2) bei Potenzialabtastungsgeschwindigkeiten zwischen 1 mV.s⁻¹ und 10000 mV.s⁻¹, vorzugsweise zwischen 10 mV.s⁻¹ und 1000 mV.s⁻¹, bevorzugter zwischen 10 mV.s⁻¹ und 100 mV.s⁻¹,
wobei das Verfahren vor Schritt b) einen Schritt b1) der Potenzialabtastung bei einer zyklischen Voltamperometrie zwischen +0,1 V und +1,5 V unter Verwendung einer Ag/AgCl-Referenzelektrode bei Potenzialabtastungsgeschwindigkeiten zwischen 1 mV.s⁻¹ und 10000 mV.s⁻¹, vorzugsweise zwischen 10 mV.s⁻¹ und 1000 mV.s⁻¹, bevorzugt zwischen 10 mV.s⁻¹ und 100 mV.s⁻ 1, ohne Messung der Stromänderung umfasst.

2. Verfahren nach Anspruch 1, **dadurch gekennzeichnet, dass** in Schritt b) die Potenzialabtastung durch zyklische Voltamperometrie zwischen +0,1 V und +1,9 V, vorzugsweise zwischen +0,1 V und +1,5 V, durchgeführt wird.

3. Verfahren nach Anspruch 1 oder 2, **dadurch gekennzeichnet, dass** die Nahrungsmittelflüssigkeit Wein ist.

4. Verfahren nach einem der vorhergehenden Ansprüche, **dadurch gekennzeichnet, dass** die Messelektrode ein Verhältnis R_{théo} zwischen 3 und 100, vorzugsweise zwischen 3 und 20, aufweist.

5. Verfahren nach einem der vorhergehenden Ansprüche, **dadurch gekennzeichnet, dass** es weiterhin umfasst:
- vor Schritt b) einen Schritt b2) der Erstellung eines Diagramms, das den pH-Wert der Nahrungsmittelflüssigkeit in Abhängigkeit von der Position des Reduktionspeaks der Goldoxide bei Potenzialabtastungen durch zyklische Voltamperometrie angibt, die in der Nahrungsmittelflüssigkeit bei verschiedenen pH-Werten unter Verwendung einer Ag/AgCl-Referenzelektrode bei Potenzialabtastungsgeschwindigkeiten zwischen 1 mV.s⁻¹ und 1000 mV.s⁻¹, vorzugsweise zwischen 10 mV.s⁻¹ und 1000 mV.s⁻¹, bevorzugt zwischen 10 mV.s⁻¹ und 100 mV.s⁻¹, durchgeführt werden,
- das Bestimmen des Alkoholgehalts, Vol., der Nahrungsmittelflüssigkeit, und
- während Schritt b) das Messen der Temperatur T der Nahrungsmittelflüssigkeit, wodurch der Prozentsatz des molekularen SO₂ in dem nicht komplexierten SO₂ mithilfe der folgenden Formel erhalten wird: $% {SO}_{2} molekular = 100 / \left[{10}^{\left(pH-pK1\right)}+1\right]$ wobei ${pK}_{1} = 1 , 949 + \left(T-20\right) \times 0 , 0322 + \left(TAV-10\right) \times 0 , 01971$

6. Elektrochemische Vorrichtung zum Nachweis und/oder zur Quantifizierung des freien SO₂ in einer wässrigen oder hydroalkoholischen Nahrungsmittelflüssigkeit, wobei die Vorrichtung so konfiguriert ist, dass sie die Schritte des Verfahrens nach einem der Ansprüche 1 bis 5 durchführt, wobei die Vorrichtung umfasst:
- eine Referenzelektrode (2)
- eine Gegenelektrode (3)
- eine Messelektrode (1), deren aktive Oberfläche vollständig aus Gold besteht, wobei diese Messelektrode ein Verhältnis R_{théo} zwischen ihrer Abwicklungsfläche (A_{dev}) und ihrer Ansichtsfläche (Aₐₚₚ) größer oder gleich 3, vorzugsweise zwischen 3 und 100, noch bevorzugter zwischen 3 und 20, aufweist, und
- eine Vorrichtung zur Durchführung einer zyklischen Voltamperometrie mit einer Potenzialabtastung zwischen -1 V und +2 V unter Verwendung einer Ag/AgCl-Referenzelektrode bei Potenzialabtastungsgeschwindigkeiten zwischen 1 mV.s⁻¹ und 10000 mV.s⁻¹, vorzugsweise zwischen 10 mV.s⁻¹ und 1000 mV.s⁻¹, noch bevorzugter zwischen 10 mV.s⁻¹ und 100 mV.s⁻¹.

7. Vorrichtung nach Anspruch 6, **dadurch gekennzeichnet, dass** die Vorrichtung die Durchführung einer zyklischen Voltamperometrie mit einer Potenzialabtastung zwischen +0,1 V und +1,9 V, vorzugsweise zwischen +0,1 V und +1,5 V, ermöglicht.

8. Vorrichtung nach Anspruch 6 oder 7, **dadurch gekennzeichnet, dass** die Messelektrode eine Elektrode ist, die einen mit einer porösen Goldhülle beschichteten Träger umfasst, wobei diese Hülle eine Dicke und eine Porengröße aufweist, so dass die Messelektrode ein Verhältnis R_{théo} zwischen ihrer Abwicklungsfläche (A_{dev}) und ihrer Ansichtsfläche (Aₐₚₚ) größer oder gleich 3, vorzugsweise zwischen 3 und 100, noch bevorzugter zwischen 3 und 20, aufweist.

9. Vorrichtung nach Anspruch 6 oder 7, **dadurch gekennzeichnet, dass** die Messelektrode eine Elektrode ist, die einen mit einer rauen Goldschicht beschichteten Träger umfasst, die aus einem Netz von Goldkristalliten nano- bis mikrometrischer Größe besteht, so dass die Messelektrode ein Verhältnis R_{théo} zwischen ihrer Abwicklungsfläche (A_{dev}) und ihrer Ansichtsfläche (Aₐₚₚ) größer oder gleich 3, vorzugsweise zwischen 3 und 100, noch bevorzugter zwischen 3 und 20, aufweist.

## Claims

1. Electrochemical method for detecting and/or quantifying sulphur dioxide (SO₂) in its non-complexed, so-called free, forms in an aqueous or hydroalcoholic food liquid, **characterised in that** it comprises:
a) introducing into the food liquid (4) a measuring electrode (1) whose active surface in contact with the food liquid is made entirely of gold, this measuring electrode having a ratio Rₜₕₑₒ between its developed area (A_{dev}) and its apparent area (Aₐₚₚ) that is greater than or equal to 3,
b) measuring the change in current produced by the oxidation of free SO₂, present in the food liquid during a potential scan by cyclic voltamperometry carried out between -1 V and +2 V, using an Ag/AgCl reference electrode (2), at potential scan rates ranging from 1 mV.s ⁻¹ to 10000 mV.s -1, preferably between 10 mV.s - 1 and 1000 mV.s ⁻¹, more preferably between 10 mV.s ⁻¹ and 100 mV.s ⁻¹,
said method comprises, prior to step b), a step b1) involving a potential scan during cyclic voltamperometry, performed between +0.1 V and +1.5 V, using an Ag/AgCl reference electrode, at potential scan rates ranging from 1 mV.s ⁻¹ to 10000 mV.s ⁻¹, preferably between 10 mV.s ⁻¹ and 1000 mV.s ⁻¹, preferably between 10 mV.s ⁻¹ and 100 mV.s ⁻ 1, without measuring the change in current.

2. Method according to claim 1, **characterised in that**, in step b), the potential scan by cyclic voltamperometry is carried out between +0.1 V and +1.9 V, preferably between +0.1 V and +1.5 V.

3. Method according to claim 1 or 2, **characterised in that** the food liquid is wine.

4. Method according to any one of the preceding claims, **characterised in that** the measuring electrode has a ratio Rₜₕₑₒ of between 3 and 100, preferably between 3 and 20.

5. Method according to any one of the preceding claims, **characterised in that** it further comprises:
- before step b), a step b2) of establishing a chart giving the pH value of the food liquid as a function of the position of the gold oxides reduction peak during potential scans by cyclic voltamperometry carried out in said food liquid at different pH, using an Ag/AgCl reference electrode, at potential scan rates between 1 mV.s ⁻¹ and 1000 mV.s ⁻¹, preferably between 10 mV.s ⁻¹ and 1000 mV.s ⁻¹, preferably between 10 mV.s ⁻¹ and 100 mV.s ⁻¹,
- determining the alcoholic strength, TAV, of the food liquid, and
- during step b) measuring the temperature T of the food liquid, which allows the percentage of molecular SO₂ contained in the non-complexed SO₂ to be determined using the following formula: $% molecular {SO}_{2} = 100 / \left[{10}^{\left(pH-pK1\right)}+1\right]$ where ${pK}_{1} = 1.949 + \left(T-20\right) \times 0.0322 + \left(TAV-10\right) \times 0.01971 .$

6. Electrochemical device for detecting and/or quantifying free SO₂ in an aqueous or hydroalcoholic food liquid, said device being configured to implement the steps of the method according to any one of claims 1 to 5, said device comprising:
- a reference electrode (2),
- a counter-electrode (3),
- a measuring electrode (1) whose active surface is entirely made of gold, this measuring electrode having a ratio Rₜₕₑₒ between its developed area (A_{dev}) and its apparent area (Aₐₚₚ) that is greater than or equal to 3, preferably between 3 and 100, more preferably between 3 and 20, and
- a device capable of performing cyclic voltamperometry with a potential scan ranging between -1 V and +2 V, using an Ag/AgCl reference electrode, at potential scan rates of between 1 mV.s ⁻¹ and 10000 mV.s ⁻¹, preferably between 10 mV.s⁻¹ and 1000 mV.s ⁻¹, more preferably between 10 mV.s ⁻¹ and 100 mV.s ⁻¹.

7. Device according to claim 6, **characterised in that** the device enables cyclic voltamperometry to be performed with a potential scan ranging between +0.1 V and +1.9 V, preferably between +0.1 V and +1.5 V.

8. Device according to claim 6 or 7, **characterised in that** the measuring electrode is an electrode comprising a substrate covered with a porous gold sheath, wherein the thickness and pore size of said sheath are such that the measuring electrode has a ratio Rₜₕₑₒ between its developed area (A_{dev}) and its apparent area (Aₐₚₚ) that is greater than or equal to 3, preferably between 3 and 100, more preferably between 3 and 20.

9. Device according to claim 6 or 7, **characterised in that** the measuring electrode is an electrode comprising a substrate covered with a rough gold layer formed by a network of gold crystallites ranging in size from nanometers to micrometers, such that the measuring electrode has a ratio Rₜₕₑₒ between its developed area (A_{dev}) and its apparent area (Aₐₚₚ) that is greater than or equal to 3, preferably between 3 and 100, more preferably between 3 and 20.
